(19) Europäisches Patentamt
European Patent Office
Office européen des brevets

(11) Veröffentlichungsnummer: **0 474 016 B1**

(12) **EUROPÄISCHE PATENTSCHRIFT**

(45) Veröffentlichungstag der Patentschrift: **19.07.95**

(51) Int. Cl.[6]: **C07D 307/79**, C07C 49/825, C07C 39/27

(21) Anmeldenummer: **91113880.8**

(22) Anmeldetag: **20.08.91**

# (54) Verfahren zur Herstellung von 2,3-Dihydrobenzofuranen.

(30) Priorität: **31.08.90 DE 4027573**

(43) Veröffentlichungstag der Anmeldung:
**11.03.92 Patentblatt 92/11**

(45) Bekanntmachung des Hinweises auf die Patenterteilung:
**19.07.95 Patentblatt 95/29**

(84) Benannte Vertragsstaaten:
**CH DE DK FR GB IT LI NL**

(56) Entgegenhaltungen:
**EP-A- 0 345 593**
**US-A- 4 857 516**

**CHEMICAL ABSTRACTS, Band 102, Nr. 21, 27. Mai 1985, Columbus, Ohio, USAARDUINI, ARTURO et al. "Selective synthesis of 2-al- kenylphenols and 2,2-dialkyl- -2,3-dihydrobenzofurans from 2-hydroxybenzyl alcohols." Seite 565,Spalte 2, Zusammenfassung-Nr. 184 932j**

(73) Patentinhaber: **BASF Aktiengesellschaft**
**Carl-Bosch-Strasse 38**
**D-67063 Ludwigshafen (DE)**

(72) Erfinder: **Harreus, Albrecht, Dr.**
**Teichgasse 13**
**W-6700 Ludwigshafen (DE)**
Erfinder: **Wolf, Bernd, Dr.**
**Halbergstrasse 4**
**W-6701 Fussgoenheim (DE)**
Erfinder: **Wild, Jochen, Dr.**
**St.-Martin-Strasse 22**
**W-6701 Ruppertsberg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 99, Nr. 19, 7. November 1983, Columbus, Ohio, USACAS-NATI, GUISEPPE et al. "O-Ouinonemethide interme- diates and their role incoordinated reactions of magnesium phenoxides with alpha-branched aliphaticaldehydes." Seite 538, Spalte 1, Zusammenfassung-Nr. 157 477c

CHEMICAL ABSTRACTS, Band 73, Nr. 17, 26. Oktober 1970, Columbus, Ohio, USAMARTINI, JOSEPH C. et al. "New preparation of coumarans." Seite 339, Spalte2, Zusammenfassung-Nr. 87 706p

CHEMICAL ABSTRACTS, Band 112, Nr. 7, 12. Februar 1990, Columbus, Ohio, USALAAN, JAN A. M. et al. "The use of aluminum phenolate in ortho-alkylation ofphenol and hydroquinone with alka- dienes: formation of cyclic ethers." Seite733, Spalte 1, Zusammenfassung-Nr. 55 497d

CHEMICAL ABSTRACTS, Band 92, Nr. 5, 4. Februar 1980, Columbus, Ohio, USAGER-VAIS, C. et al. "Cyclode- hydration of alcoholic by HMPA. A new route todihydro- benzofurans and dihydroben- zopyrans." Seite 758, Spalte 1,Zusammenfassung-Nr. 41 667r

## Beschreibung

Die vorliegende Erfindung betrifft ein neues Verfahren zur Herstellung von 2,2-disubstituierten 2,3-Dihydrobenzofuranen I

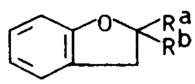

I

und deren Derivaten, wobei $R^a$ und $R^b$ unabhängig voneinander für C-organische Reste stehen, durch Umsetzung von 2-Hydroxybenzylalkoholen II bzw. deren Derivaten

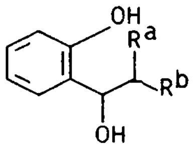

II

in einem inerten organischen Lösungsmittel in Gegenwart eines sauren Ionenaustauscherharzes.

Die Herstellung von Dihydrobenzofuranen ist prinzipiell bekannt. Am häufigsten wird der Aufbau des Heterocyclus mittels Claisen-Umlagerung von o-Allylphenylethern und anschließender Cyclisierung der erhaltenen ortho- Allylphenole vorgenommen. (Houben-Weyl, Methoden der organischen Chemie, Band 6/3, Seite 620 ff (1965); DE-OS 2108932). Letztere Reaktionen weisen jedoch zur Gewinnung der substituierten Dihydrobenzofurane der Formel I wesentliche Nachteile auf, vor allem die mangelhafte Regioselektivität und die schwere Zugänglichkeit der benötigten Allylierungsreagenzien.

Außerdem ist bekannt, daß man 2,2-disubstituierte 2,3-Dihydrobenzofurane durch Umsetzung der entsprechenden 2-Hydroxybenzylalkohole in Toluol in Gegenwart katalytischer Mengen Amberlyst® 15, einem sauren Ionenaustauscherharz mit Sulfonsäuregruppen, erhält (Arduini et al., Synthesis, 1984, S. 950 f.).

Die beschriebene Synthese in Gegenwart von sauren Ionenaustauschern ist für eine wirtschaftliche industrielle Anwendung jedoch ungeeignet, da wegen der Gefahr der Polymerisation von Edukten und Zwischenprodukten die Umsetzung in hohen Verdünnungen durchgeführt werden muß. Zusätzlich zu dem dadurch auftretenden Problem der Aufbereitung bzw. Entsorgung großer Mengen an Lösungsmittel und dem Problem des hohen Energieverbrauchs werden nach diesem Verfahren nur geringe Raum-Zeit-Ausbeuten erzielt.

Wie bereits aus der Literatur bekannt ist, verläuft die Cyclisierung der 2-Hydroxybenzylalkohole in zwei Stufen:

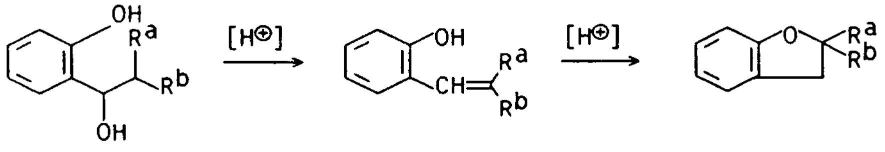


II V I

Wie aus dem Reaktionsschema ersichtlich, wird sowohl die Eliminierung (1. Stufe) als auch die Cyclisierung (2. Stufe) durch Protonen katalysiert. Da sowohl der Hydroxybenzylalkohol II als auch das intermediär gebildete 2-Vinylphenol V untereinander und mit sich selbst Polymerisate bilden können, führen lange Reaktionszeiten und hohe Konzentrationen an diesen Stoffen im Reaktionsmedium zu Nebenreaktionen und damit zu Ausbeuteverlusten bezüglich des 2,3-Dihydrobenzofurans I.

Aufgabe der vorliegenden Erfindung war ein wirtschaftlich anwendbares Verfahren zur Herstellung von 2,2-disubstituierten 2,3-Dihydrobenzofuranen.

Dementsprechend wurde ein Verfahren zur Herstellung von 2,2-disubstituierten 2,3-Dihydrobenzofuranen I

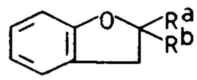

I

und deren Derivaten, wobei $R^a$ und $R^b$ die vorstehend gegebene Bedeutung haben, durch Umsetzung von 2-Hydroxybenzylalkoholen II bzw. deren Derivaten

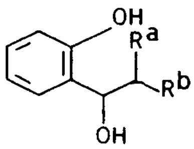

II

in einem inerten organischen Lösungsmittel in Gegenwart eines sauren Ionenaustauscherharzes gefunden, welches dadurch gekennzeichnet ist, daß man die Umsetzung in Gegenwart von inerten, Wasser entziehenden Stoffen durchführt.

Das erfindungsgemäße Verfahren beruht darauf, daß durch Zugabe eines inerten, Wasser entziehenden Stoffes der Hydroxybenzylalkohol II schneller in das 2-Vinylphenol V umgewandelt wird. Auf diesem Wege wird die Reaktionszeit und damit auch die Wahrscheinlichkeit der Nebenreaktionen herabgesetzt.

Außerdem wird die Umsetzungsgeschwindigkeit von der Menge des eingesetzten sauren Katalysators beeinflußt. Das optimale Verhältnis von Katalysator zu Hydroxybenzylalkohol II ist dabei von der Art und der Position der Substituenten im Phenylteil des Benzylalkohols II abhängig und muß von Fall zu Fall ermittelt werden.

Bereits in der in Synthesis, 1984 veröffentlichten Arbeit wurde die Eignung von Kieselgel sowie von saurem bzw. neutralem Aluminiumoxid als Katalysatoren der Cyclisierung untersucht. Obwohl die genannten Stoffe sowohl saure (bzw. im letztgenannten Fall neutrale) als auch Wasser entziehende Eigenschaften vereinen, erzielten die Autoren mit diesen Katalysatoren weniger als 50 % Ausbeute.

Werden die Alkohole der Formel II bzw. der von Arduini et al. verwendete Benzylalkohol III unter gleich hohen Konzentrationsbedingungen jedoch ohne Trockenmittelzusatz umgesetzt, so kommt es nur zu einem Teilumsatz der Alkohole II bzw. bleibt die Reaktion auf der Zwischenstufe der Olefine stehen und zusätzliche Nebenreaktionen laufen ab.

Als erfindungsgemäß für die Cyclisierung erforderliche inerte, Wasser entziehende Stoffe eignen sich besonders anorganische Trockenmittel wie Magnesiumsulfat, Natriumsulfat, Caliumsulfat wasserfrei (beispielsweise Drierite®), Calciumchlorid, bevorzugt Molekularsiebe mit einer Porengröße von 3 Å bis 10 Å, besonders bevorzugt 3 bis 4 Å. Diese Trockenmittel werden im allgemeinen mindestens in solchen Mengen verwendet, in denen sie entsprechend ihrer Aufnahmekapazität für Wasser ein mol Wasser pro mol 2-Hydroxybenzylalkohol aufnehmen können.

Die Maximalmenge an Trockenmittel wird durch verfahrenstechnische und wirtschaftliche Erwägungen bestimmt; ein Optimum muß unter Berücksichtigung der folgenden Punkte von Fall zu Fall gefunden werden:

- Die Geschwindigkeit des Entzugs von Wasser steht in direktem Zusammenhang zur Oberfläche des Trockenmittels und ist damit direkt von der Menge und der Körnung des Trockenmittels abhängig.
- Da die Trockenmittel als feste Phase im Reaktionsmedium vorliegen, ist darauf zu achten, daß sie eine ausreichende Konvektion der Reaktionsmischung nicht behindern.
- Da die Trockenmittel vorzugsweise eine große Oberfläche haben, ist darauf zu achten, daß Ausbeuteverluste bei der Aufarbeitung, die beispielsweise durch die Einlagerung des Produkts in den Trockenmittelrückstand auftreten können, vermieden oder zumindest minimiert werden.
- In Fällen, in denen die Trockenmittel nicht ohne Verlust regeneriert werden können, könnten wirtschaftliche Aspekte zu berücksichtigen sein.

Für den Fall, daß man als Trockenmittel Molekularsiebe verwendet, haben sich folgende Punkte als vorteilhaft erwiesen:

- Das Molekularsieb sollte eine Porengröße von mindestens 3 Å und nicht mehr als 10 Å, insbesondere 3 Å bis 4 Å haben.
- Die Menge an Molekularsieb mit 3 Å bis 4 Å sollte entsprechend den vorstehend ausgeführten Erwägungen zwischen 50 g und 500 g, insbesondere 100 g bis 300 g pro mol 2-Hydroxybenzylalkohol liegen.

Die Konzentration des 2-Hydroxybenzylalkohols im Reaktionsmedium ist nach dem erfindungsgemäßen Verfahren in weiten Bereichen variabel; auch hier muß das Optimum unter Berücksichtigung verschiedener

Gesichtspunkte abgewägt werden:

- Die Lösungsmittelmenge muß mindestens ausreichend sein, um den eingesetzten 2-Hydroxybenzylalkohol zu lösen.
- Außerdem ist die Lösungsmittelmenge abhängig von der mindestens zu verwendenden Menge an Trockenmittel und Ionenaustauscher, die Konvektion des Reaktionsmediums muß gewährleistet sein.
- In Fällen, in denen die Lösungsmittel nicht ohne Verlust regeneriert werden können, könnten wirtschaftliche Aspekte zu berücksichtigen sein.

Im allgemeinen verwendet man das Lösungsmittel in Mengen von 0,5 l bis 10 l pro mol des 2-Hydroxybenzylalkohols II (entsprechend 0,1 bis 2 mol/l an II), insbesondere 1 l bis 3 l pro mol II (entsprechend 0,3 bis 1 mol/l an II).

Im Hinblick darauf, daß zweckmäßigerweise möglichst mit geringen Mengen an Lösungsmittel gearbeitet wird, ist es empfehlenswert, die minimal benötigte Menge an Lösungsmittel gemeinsam mit den erforderlichen Mengen an Katalysator und Trockenmittel vorzulegen und den 2-Hydroxybenzylalkohol II sukzessive zuzugeben. Diese Art der Reaktionsführung ist außerdem geeignet, die Gefahr der Polymerisation der Edukte herabzusetzen bzw. die Polymerisation vollständig zu vermeiden (Verdünnungsprinzip).

Als inerte organische Lösungsmittel finden aliphatische und aromatische, gegebenenfalls halogenierte Kohlenwasserstoffe wie Dichlormethan, Chloroform, Tetrachlorkohlenstoff, Cyclohexan, Benzol, Toluol, Xylol bzw. Mischungen derselben, bevorzugt Toluol oder Benzol Verwendung.

Als Katalysatoren eignen sich generell die stark sauren Ionenaustauscherharze, als Beispiele hierfür seien das eingangs erwähnte Amberlyst® 15, aber auch andere Amberlyst®-Arten sowie Lewatit® und Amberlite® genannt.

Die Menge an zu verwendenden Katalysator ist im wesentlichen von dem Grad der Beladung dieses Katalysators mit Protonen, der Menge an 2-Hydroxybenzylalkohol II und von der Art der Substituenten im Phenylteil dieses Alkohols abhängig.

Üblicherweise werden pro mol des Alkohols 0,001 bis 1,0 mol-Äq. Protonen, vorzugsweise 0,01 bis 0,5 mol-Äq. Protonen eingesetzt.

Die Umsetzungstemperatur kann in weiten Bereichen zwischen der Raumtemperatur (25 °C) und dem Siedepunkt des Lösungsmittels bzw. des Lösungsmittelgemisches variiert werden. Die maximale Temperatur richtet sich im allgemeinen nach der Stabilität der Vor- bzw. Zwischenprodukte (Verbindungen II und V).

Üblicherweise erfolgt die Umsetzung bei 25 °C mit ausreichender Geschwindigkeit, während die Verbindungen im allgemeinen bis zu Temperaturen von 180 °C belastet werden können, ohne daß spontane Zersetzung auftritt. Vorzugsweise wird die Umsetzung bei Temperaturen von 30 °C bis 150 °C, insbesondere 60 °C bis 120 °C durchgeführt.

Die Aufarbeitung der Reaktionsmischung und die Isolierung des Produktes erfolgt in an sich bekannter Weise, indem zunächst der Katalysator und das Trockenmittel aus der Reaktionsmischung entfernt werden und aus der so erhaltenen Reaktionslösung anschließend durch Kristallisation, Chromatographie oder Destillation das Produkt isoliert wird.

Das erfindungsgemäße Verfahren eignet sich zur Herstellung von 2,2-disubstituierten 2,3-Dihydrobenzofuranen I und substituierten Derivaten aus den entsprechenden 2-Hydroxybenzylalkoholen insbesondere solchen der allgemeinen Formel II'

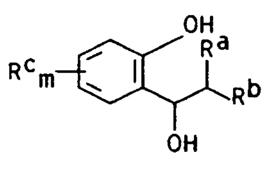

II'

in welcher die Substituenten und der Index folgende Bedeutung haben:

$R^a$ und $R^b$ unabhängig voneinander
C-organische Reste wie Alkyl- und Arylgruppen, wobei die Natur dieser Substituenten dergestalt sein muß, daß sie eine positive Teilladung an dem Kohlenstoffatom, an das sie gebunden sind, stabilisieren; gegen die Reaktionsbedingungen inerte Substituenten an diesen C-organischen Resten haben nach den bisherigen Erkenntnissen keinen Einfluß auf das Verfahren;

$R^c$ unter den Reaktionsbedingungen inerte Substituenten wie C-organische Reste wie vorstehend genannt sowie Alkenyl- und Alkinylgruppen, die direkt oder über Heteroatome wie Sauerstoff, Schwefel oder Stickstoff gebunden sein können; Halogenatome, Cyano-, Carboxyl- oder Nitrogruppen;

m 0, 1, 2 oder 3, wobei die Reste $R^c$ verschieden sein können, wenn m für 2 oder 3 steht; der Wert von m beeinflußt das Verfahren nur insoweit, als hohe sterische Hinderung durch voluminöse Reste oder Reste in 2- oder 5-Position des Phenols die Reaktionsgeschwindigkeit herabsetzen können.

Bevorzugt kommen als Reste $R^a$ und $R^b$, und $R^c$ folgende Gruppen in Betracht:
Alkylgruppen mit bis zu sechs C-Atomen, insbesondere $C_1$-$C_4$ Alkylgruppen wie Methyl, Ethyl, Propyl, 1-Methylethyl, Butyl, 1-Methylpropyl und 2-Methylpropyl;
Alkenylgruppen mit bis zu sechs C-Atomen, insbesondere $C_2$-$C_4$-Alkenylgruppen wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl und 2-Methyl-2-propenyl;
Alkinylgruppen mit bis zu sechs C-Atomen, insbesondere $C_2$-$C_4$-Alkinylgruppen wie Ethinyl, 1-Propinyl, 2-Propinyl, 1-Butinyl, 2-Butinyl, 3-Butinyl und 1-Methyl-2-propinyl;
Arylgruppen wie insbesondere Phenyl;
Die vorstehend genannten C-organischen Reste können direkt oder über Heteroatome wie Sauerstoff, Schwefel oder Stickstoff gebunden sein;
Außerdem kommen Halogenatome wie Fluor, Chlor, Brom und Jod, insbesondere Fluor, Chlor und Brom, Cyano-, Carboxyl- oder Nitrogruppen in Betracht.

Der Index m hat im allgemeinen den Wert 0, 1, 2 oder 3, vorzugsweise 0, 1 oder 2, wobei die Reste verschieden sein können, wenn m 2 oder 3 bedeutet.

Die vorstehend genannten Reste können ihrerseits durch Heteroatome wie Stickstoff, Sauerstoff unf Schwefel unterbrochen sein oder weitere inerte Reste wie Halogen, Nitro, Sulfonyl, Arylsulfonyl und Carboxyl tragen.

Die nach dem erfindungsgemäßen Verfahren besser zugänglichen 2,2-disubstituierten 2,3-Dihydrobenzofurane I und deren Derivate dienen beispielsweise als Zwischenprodukte in der Herstellung von Pharmaka, Farben und Pflanzenschutzmitteln.

Experimenteller Teil

1. Untersuchungen zur Cyclisierung der Verbindungen II zu den 2,2-disubstituierten 2,3-Dihydrobenzofuranen I

Der entsprechende 2-Hydroxybenzylalkohol (BA) II, der saure Ionenaustauscher (KAT), das Lösungsmittel (LM) und gegebenenfalls das Trockenmittel (TM) wurden entsprechend den in Tabelle 1 angegebenen Verhältnissen zusammengegeben und bei der jeweiligen Temperatur (T) gerührt.

Zur Beurteilung des Versuchsverlaufs wurden folgende Bestimmungen durchgeführt:

*) Der Reaktionsmischung wurde eine Probe von 2 ml entnommen, diese Probe über Kieselgur abfiltriert und ohne Nachwaschen das Filtrat an einer Kapillarsäule gaschromatographisch untersucht. Der Lösungsmittelanteil wurde dabei gleich 0 % gesetzt.

**) Der Reaktionsansatz wurde über Kieselgur abgesaugt, der Rückstand mit Dichlormethan nachgewaschen, das Filtrat eingeengt und das Rohprodukt gaschromatographisch an einer Kapillarsäule untersucht, zur Verdünnung der Probe wurde dabei Dichlormethan eingesetzt. Aus dem Gehalt an I und der Rohproduktauswaage wurde die nicht isolierte Ausbeute bestimmt. Zusätzlich wurde das Rohprodukt $^1H$-NMR-spektroskopisch untersucht.

Tabelle 1

| Nr. | BA II | T (°C) | LM | TM | Eing. Menge BA II (mol) | Verhältnisse der Reaktionsparameter pro 1000 ml Lösungsmittel BA II (mol) | KAT (mol $H^+$) | TM (g) |
|---|---|---|---|---|---|---|---|---|
| V-0 | i | 80 | T | - | 0,01 | 0,1 | 0,01 | - |
| V-1 | i | 80 | T | - | 0,1 | 0,1 | 0,01 | - |
| V-2 | i | 80 | T | - | 0,1 | 0,4 | 0,04 | - |
| E-1 | i | 80 | T | MS | 0,1 | 0,4 | 0,04 | 200 |
| V-3 | ii | 80 | T | - | 0,04 | 0,5 | 0,05 | - |
| E-2 | ii | 80 | T | MS | 0,04 | 0,5 | 0,05 | 200 |
| E-3 | ii | 80 | T | MS | 0,04 | 0,5 | 0,2 | 200 |
| E-5 | ii | 80 | T | $MgSO_4xH_2O$ | 0,04 | 0,5 | 0,2 | 200 |
| E-6 | ii | e | DM | MS | 0,04 | 0,5 | 0,05 | 200 |
| E-7 | ii | 25 | T | MS | 0,04 | 0,5 | 0,2 | 200 |
| E-8 | ii | 40 | T | MS | 0,04 | 0,5 | 0,2 | 200 |
| E-9 | ii | 65 | T | MS | 0,04 | 0,5 | 0,2 | 200 |

i: 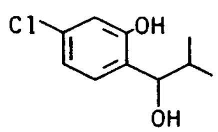

ii: 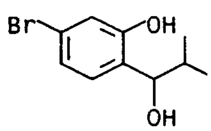

T: Toluol

DM: Dichlormethan

MS: Molekularsieb 3 Å

e: Siedepunkt der Reaktionsmischung

KAT: Amberlyst 15

Eing.: Eingesetzte

Im Vergleich zu der von Arduini et al. (Synthesis 1984, 950) angegebenen allgemeinen Arbeitsvorschrift (Methode A, o.o.A. S. 952 entspricht Tabelle 1, Versuch V-O), wurde durch Variation die Abhängigkeit des Verfahrens von der Konzentration an 5-Chlor-2-(1-hydroxymethylpropyl)-phenol (BA II) sowie vom Trockenmittelzusatz beispielhaft untersucht (Versuche V-1, V-2 und E-1 in Tabelle 1-1). Es zeigt sich, daß allein bei Verzehnfachung des Originalansatzes zur Erreichung eines vollständigen Umsatzes über 78h benötigt werden. Wird die Konzentration des Edukts um den Faktor 4 erhöht fällt die Ausbeute an I erheblich ab (Vergleich der 24 h Werte von V-1 und V-2), kann jedoch wieder sehr deutlich angehoben werden wenn beispielsweise in Gegenwart von Molekularsieb als Trockenmittel umgesetzt wird (E-1).

Tabelle 1-1

| Einfluß der Konzentration (Standardisiert auf 1000 ml Toluol; T = 80 °C; BA II = i) | | | | | |
|---|---|---|---|---|---|
| | BA II (mol) | KAT (mol $H^+$) | TM (g) | t (h) | GC-Analyse (% I) |
| V-1 | 0,1 | 0.01 | - | 24<br>78 | 84<br>92 |
| V-2<br>E-1 | 0,4<br>0,4 | 0,04<br>0,04 | -<br>200 | 24<br>24 | 61<br>77 |

Tabelle 1-2

| Einfluß des Trockenmittels (Standardisiert auf 1000 ml Toluol; T = 80 °C; BA II = ii) | | | | | |
|---|---|---|---|---|---|
| | BA II (mol) | KAT (mol $H^+$) | TM (g) | t (h) | n.i.Ausbeute** (% I) |
| V-3 | 0,5 | 0,05 | - | 7 | 52 |
| E-2 | 0,5 | 0,05 | 200 | 7 | 77 |
| E-3 | 0,5 | 0,2 | 200 | 7 | 90 |

Tabelle 1-3

| Vergleich der Lösungsmittel und der Trockenmittel (Standardisiert auf 1000 ml Lösungsmittel; 200 g TM; BA II = ii) | | | | | | | |
|---|---|---|---|---|---|---|---|
| | BA II (mol) | KAT (mol $H^+$) | TM | LM | T (°C) | t h | GC-Analyse (% I) |
| E-2 | 0,5 | 0,05 | MS | Toluol | 80 | 4<br>7 | 73<br>83 |
| E-6 | 0,5 | 0,05 | MS | DM | e | 4<br>7 | 60<br>63 |
| E-3 | 0,5 | 0,2 | MS | Toluol | 80 | 4<br>7 | 86<br>91 |
| E-5 | 0,5 | 0,2 | $MgSO_4$ $xH_2O$ | Toluol | 80 | 4<br>7 | 77<br>82 |

Tabelle 1-4

| Temperaturabhänigkeit des Verfahrens (Standardisiert auf 1000 ml Toluol; 200 g MS; BA II = ii) | | | | | |
|---|---|---|---|---|---|
| | BA II (mol) | KAT (mol $H^+$) | T (°C) | t (h) | n.i. Ausbeute** (% I) |
| E-9 | 0,5 | 0,2 | 65 | 7 | 64 |
| E-3 | 0,5 | 0,2 | 80 | 7 | 90 |

## Patentansprüche

1. Verfahren zur Herstellung von 2,2-disubstituierten 2,3-Dihydrobenzofuranen I

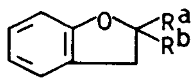

I

und deren Derivaten, wobei $R^a$ und $R^b$ unabhängig voneinander für C-organische Reste stehen, durch Umsetzung von 2-Hydroxybenzylalkoholen II bzw. deren Derivaten

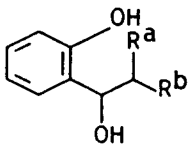

II

in einem inerten organischen Lösungsmittel in Gegenwart eines sauren Ionenaustauscherharzes, dadurch gekennzeichnet, daß man die Umsetzung in Gegenwart von inerten, Wasser entziehenden Stoffen durchführt.

2. Verfahren nach Anspruch 1, dadurch gekennzeichnet, daß man als inerte, Wasser entziehende Stoffe anorganische Trockenmittel verwendet.

3. Verfahren nach den Ansprüchen 1 und 2, dadurch gekennzeichnet, daß man als inerte, Wasser entziehende Stoffe Molekularsiebe verwendet.

4. Verfahren nach den Ansprüchen 1, 2 und 3, dadurch gekennzeichnet, daß man die inerten, Wasser entziehenden Stoffe in solchen Mengen verwendet, in denen sie entsprechend ihrer Aufnahmekapazität für Wasser mindestens ein Mol Wasser pro Mol 2-Hydroxybenzylalkohol aufnehmen können.

5. Verfahren nach Anspruch 3, dadurch gekennzeichnet, daß man 100 g bis 500 g Molekularsieb pro Mol 2-Hydroxybenzylalkohol verwendet.

6. Verfahren nach den Ansprüchen 1 bis 5, dadurch gekennzeichnet, daß man das Lösungsmittel in Mengen von 0,5 l bis 10 l pro mol 2-Hydroxybenzylalkohol verwendet.

7. Verfahren nach den Ansprüchen 1 bis 5, dadurch gekennzeichnet, daß man das Lösungsmittel in Mengen von 1 l bis 3 l pro mol 2-Hydroxybenzylalkohol II verwendet.

## Claims

1. A process for the preparation of a 2,2-disubstituted 2,3-dihydrobenzofuran I

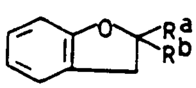

I

or a derivative thereof, where $R^a$ and $R^b$, independently of one another, are C-organic radicals, by reacting a 2-hydroxybenzyl alcohol II

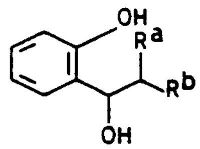

II

or a derivative thereof in an inert organic solvent in the presence of an acidic ion exchanger resin, which comprises carrying out the reaction in the presence of an inert dehydrating substance.

2. A process as claimed in claim 1, wherein the inert dehydrating substance used is an inorganic desiccant.

3. A process as claimed in claim 1 or 2, wherein the inert dehydrating substance used is a molecular sieve.

4. A process as claimed in claim 1 or 2 or 3, wherein the inert dehydrating substance is used in an amount in which, in accordance with its absorption capacity for water, it is able to absorb at least one mol of water per mol of 2-hydroxybenzyl alcohol.

5. A process as claimed in claim 3, wherein from 100 g to 500 g of molecular sieve are used per mol of 2-hydroxybenzyl alcohol.

6. A process as claimed in claim 1 or 2 or 3 or 4 or 5, wherein the solvent is used in an amount of from 0.5 l to 10 l per mol of 2-hydroxybenzyl alcohol.

7. A process as claimed in claim 1 or 2 or 3 or 4 or 5, wherein the solvent is used in an amount of from 1 l to 3 l per mol of 2-hydroxybenzyl alcohol II.

## Revendications

1. Procédé de préparation de 2,3-dihydrobenzofurannes disubstitués sur 2,2

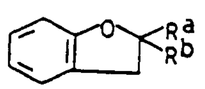

I

et leurs dérivés, $R^a$ et $R^b$ étant mis, indépendamment l'un de l'autre, pour des restes organiques en C, par réaction d'alcools 2-hydroxybenzyliques II ou leurs dérivés

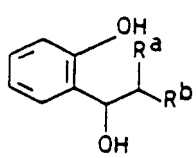

II

dans un solvant organique inerte, en présence d'une résine échangeuse d'ions acide, caractérisé par le fait que l'on effectue la réaction en présence de matières inertes, extrayant l'eau.

2. Procédé selon la revendication 1, caractérisé par le fait que l'on utilise, comme matières inertes, extrayant l'eau, des desséchants inorganiques.

3. Procédé selon les revendications 1 et 2, caractérisé par le fait que l'on utilise comme matières inertes, extrayant l'eau, des cribles moléculaires.

**4.** Procédé selon les revendications 1, 2 et 3, caractérisé par le fait que l'on utilise les matières inertes, extrayant l'eau, en quantités telles qu'elles peuvent absorber au moins une mole d'eau par mole d'alcool 2-hydroxybenzylique, selon leur capacité d'absorption de l'eau.

**5.** Procédé selon la revendication 3, caractérisé par le fait que l'on utilise 100 g à 500 g de crible moléculaire par mole d'alcool 2-hydroxybenzylique.

**6.** Procédé selon les revendications 1 à 5, caractérisé par le fait que l'on utilise le solvant en quantités de 0,5 litre à 10 litres par mole d'alcool 2-hydroxybenzylique.

**7.** Procédé selon les revendications 1 à 5, caractérisé par le fait que l'on utilise le solvant en quantités de 1 litre à 3 litres par mole d'alcool 2-hydroxybenzylique.